(19) Europäisches Patentamt
European Patent Office
Office européen des brevets

(11) Publication number: **0 084 953**
**B1**

(12) **EUROPEAN PATENT SPECIFICATION**

(45) Date of publication of patent specification: **11.05.88**

(51) Int. Cl.[4]: **C 12 P 21/02,** C 07 K 15/26, A 61 K 31/505 // A61K45/02

(21) Application number: **83300269.4**

(22) Date of filing: **19.01.83**

(54) **Interferon induction method.**

(30) Priority: **19.01.82 BG 55032/82**

(43) Date of publication of application:
**03.08.83 Bulletin 83/31**

(45) Publication of the grant of the patent:
**11.05.88 Bulletin 88/19**

(84) Designated Contracting States:
**BE CH DE FR GB IT LI SE**

(56) References cited:
**GB-A-1 492 425**

**CHEMICAL ABSTRACTS, vol. 87, no. 13, 26th September 1977, page 29, no. 95461j, Columbus, Ohio, USA M. TONEW et al.: "The antiviral activity of dipyridamole"**

**CHEMICAL ABSTRACTS, vol. 97, no. 11, 13th September 1982, page 42, no. 84825m, Columbus, Ohio, USA A. GALABOV et al.: "Dipyridamole is an interferon inducer"**

(73) Proprietor: **SO "PHARMACHIM"**
**16, Iliensko Chaussee**
**Sofia (BG)**

(72) Inventor: **Galabov, Angel Simeonov**
**7 K. Sarafov Street**
**Sofia (BG)**
Inventor: **Mastikova, Margarita Hristova**
**Komplex Serdika Block 25-4**
**Sofia (BG)**

(74) Representative: **Silverman, Warren et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 084 953 B1**

Courier Press, Leamington Spa, England.

**Description**

This invention relates to a method for the induction of interferon manufacture which is applicable both *in vitro* and *in vivo*, the interferon thus produced being effective in human and veterinary medicine especially for the prophylaxy and treatment of viral infections.

A wide variety of interferon induction methods have hitherto been proposed which make use of viruses, glycolipids of enterobacteria and other bacterial endotoxins, double-stranded phage RNA (double-stranded RNA of phage f2 of Escherichia coli, statolon), synthetic double-stranded polyribonucleotides (poly I:C, poly G:C, poly A:U, etc), certain polyanions (for example pyran, polyacrylic, polymethacrylic and polyacetal carboxylic acids) and some synthetic low molecular weight substances: tilorone hydrochloride and other fluorene products; cationic dyestuffs (toluidine blue, methylene blue, trypaflavine, acridine orange, mepacrine dihydrochloride, acraynl dihydrochloride, etc.); N,N-dioctadecyl-N'-N'-bis(2-hydroxyethyl)-propanediamine (CP—20961); 4-(3-dimethylaminoproplyamino)-1,3-dimethyl-1H-pyrazolo-[3,4-b] quinoline dihydrochloride (BL—20803), its 7-methyl derivative (BL—3849A) and other pyrazolo [3,4-b] quinolines; bis-piperidinylacetyl-dibenzofuran hydrochloride (MA—56); S-(2-aminoethyl)-isothiouronium and related compounds; 2-amino-5-bromo-6-methyl-4-pyrimidinol (U—25,166) and related compounds; and 3-(5,7-dimethyl-4-oxo-2-hydroxy-6,8-decadienyl)-glutarimide (9-methylstreptimidone) and related substances. The use of these various compounds is disclosed in various of the following documents:

(1) Borecky, L. "Virusy, imunita a inteferon", Osveta, Bratislava (1977);
(2) Sadikov, A.S., Ershov, F.I., Novohatskii, A.S., Aslanov, N.A., Auelbekov S.A. — "Induktory Interferona", FAN, Tashkent, 1978;
(3) Stewart II, W.E., in: "The Interferon System", Springer-Verlag, Vienna-New York (1979);
(4) Ershov, F., A.S. Novohatskii — "Interferon i ego Induktory", Medicina, Moskva (1980);
(5) Solovyev, V.D., T.A. Bektemirov — "Interferon v Teorii i Praktike Mediciny", Medicina, Moskva (1981);
(6) Nossik, N.N., Ershov, F.I. "Antibiotiki" 9, 695—710(1980);
(7) Wierenga, W., Skulnick, H.I., Weed, S.D., Stringfellow, D.A. in: Curr. Chemother. and Infect. Dis. — Proc. 11th Intern. Congress Chemother. and 11th Intersc. Conf. Antimicrob. Agents and Chemother., vol. 2, Boston, pp 1402—1404 (1980).

It is well-known that the interferon inducers find application: (a) as a means of producing exogenous interferon (Sendai virus and Newcastle disease virus) — a substance of protein nature used in the prophylaxis and treatment of viral infections and some oncologic diseases (see the aforementioned references (1), (3) and (6) and also:
(8) Cantell, K. in: Interferon 1979. Academic Press, London-New York, pp. 1—28 (1979);
(b) as antiviral means themselves; this is the case with double-stranded RNA of phage f2 of E. coli, poly G:C, gossipol (polyphenol isolated from cotton oil), and others [see the aforementioned references (1), (2), (4), (5)].

Of the known interferon inducers (both natural substances and synthetic products) only the double-stranded RNA of phage f2 of E.coli [the aforementioned references (1), (2), (4), (5)] and gossipol [the aforementioned references (2), (4), (5)] have so far found clinical application. These have been applied only locally (unguents and other local forms) in the treatment of viral infections caused by herpes viruses. There is evidence of limited studies carried out on volunteers to determine the efficiency of propanediamine (CP—20,961) applied locally in rhinoviral infections and there are some controversial data about the efficiency of some synthetic polyribonucleotides (poly I:C, poly G:C, poly A:U) in local application mainly in treatment of herpes keratoconjuctivitis (see the aforementioned references (2), (4) and (6) and also:
(9) Douglas, R.G. Jr., R.F. Betts in: Progress Chemother. — Proc. 8th Intern. Congress Chemother. Athens, 1979, vol. 2. Hellenic Soc. Chemother., Athens, pp 1001—1004 (1974);
(10) Kasparov, A.A., L.L. Fadeeva, S.E. Avetistov, Antibiotiki 4, 452—462 (1974);
(11) Kaufman, H.E. J. Infect. Diseases 133, 96—100 (1976).

The latter, and especially poly I:C prove to be toxic internally. Experimentally (*in vivo*), the highly active low-molecular weight interferon inducer, tilorone hydrochloride has also proved toxic and unsuitable for clinical application [see the aforementioned references (1) to (6)]. The application of inactivated viruses as inducers for prophylaxis of viral infections is not appropriate as the introduction of large virus doses as required for stimulating the interferon production, and this is far from being unharmful. The application of live vaccine viral strains also conceals many disadvantages: antiviral activity for only a short time, need for frequent introduction of a series of antigenously different infectious viral strains, possible reduction of organism resistance to infectious agents and unfavourable effect of antibody formation [see reference (6)]. It has become clear that at present there is no efficient endogenous interferon inducer for internal application which would greatly increase the indications for using this basic approach for stimulation of the non-specific organism resistance to viral infections.

Only two viruses have found application so far as inducers of production of exogenous interferon: Sendai virus and the Newcastle disease virus. However the application of synthetic inducers would offer the opportunity to eliminate the use of an infectious inducer virus.

It is an object of this invention to provide a method for the induction of interferon guaranteeing the production of high titres of the interferon in the organism and which is also applicable to the production of

interferon *in vitro.*

According to the present invention, there is provided a method of inducing the production of interferon by cellular material which is characterised by administering the compound dipyridamole to cellular material held *in vitro* and on which dipyridamole does not have a biocidal effect, retaining the dipyridamole in contact with the cellular material for a period of time sufficient for interferon production to be induced and isolating interferon from the medium in which it had been produced.

According to another aspect of the invention, there is provided a method for the production of interferon which comprises administering the compound dipyridamole in an interferon inducing single dose to a non-human warm-blooded animal, removing serum from the animal and separating interferon from the serum.

In a further aspect of the invention, there is provided the use of the compound diphridamole for the manufacture of a medicament for the induction of interferon in a warm-blooded animal to have an antiviral effect therein.

The compound dipyridamole is a known substance whose full name is 2,6-bis[di(2-hydroxyethyl)-amino]-4,8-dipiperidinopyridimo[5,4-d]pyrimidine and possesses the formula

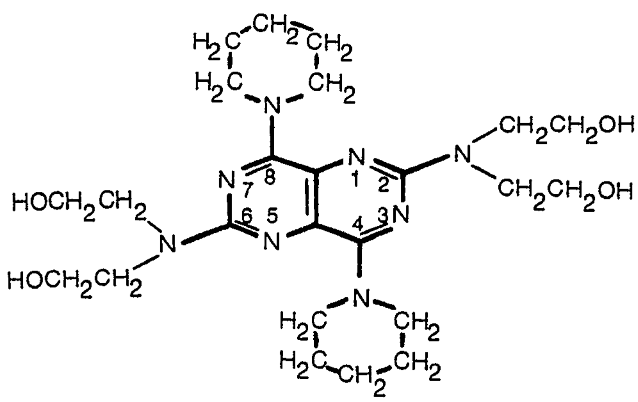

This compound has been employed medicinally as a coronary vasodilator and antiaggregant. Its antiviral effect in *in vitro* testing (in cell cultures) towards some DNA and RNA containing viruses is also known. In contrast, in the method of this invention, dipyridamole is not itself used medicinally but as a means of inducing the production of interferon which is a substance having·therapeutic activity.

The interferon-inducing activity of dipyridamole has been established *in vivo* in lymphoid cell cultures (explanted, cell lines) and non-lymphoid cells (primary cultures, cell lines and strains) from humans, mice, etc., and *in vivo* in white mice and people.

In essence, two methods are employed for characterising the dipyridamole interferon-inducing activity *in vitro*. In the first method, dipyridamole is added to a lymphoid cell suspension culture containing explanted mouse peritoneal leucocytes and prepared after Lackovič et al. ($2.5 \times 10^6$ cells in 1 ml medium — medium 199 plus 10% heated calf serum) immediately after the preparation of the culture; interferon titration samples are taken after cell incubation for 20 hours at 37°C. In the second method, monolayer cell cultures (L cells, mouse embryonal fibroblasts, human diploid embryonal pulmonary fibroblasts) are employed, the interferon titre and the antiviral resistance state being determined simultaneously. The cells, preferably cultivated for a longer time of 96 hours, are treated with dipyridamole for 4 hours at 37°C (in medium 199 to which 10% heated calf serum has been added), after which they are washed twice. Fresh medium (199 plus 2% heated calf serum), containing dipyridamole, is added and the cultures are incubated for 24 hours at 37°C. After checking the cytotoxicity of the compound microscopically, the culture medium is collected for the interferon titre measurement, and the cells are washed using Hanks' saline and inoculated by 100 $TCID_{50}$ of the vesicular stomatitis virus (Indiana strain). The virus cytopathic effect and the infectious virus yield are recorded following an incubation for 48 hours at 37°C.

To characterize the interferon-inducing activity of dipyridamole *in vivo*, the compound is administered once to female white mice weighing 20—25 g using different administration routes: intravenous, intraperitoneal or oral administration. Serum samples for measuring the interferon titre are taken at the 6th, 12th, 24th, 48th, 72nd and 96th hour (pooled serum samples from minimum 8 animals). The control is made up of a group of animals not treated with dipyridamole. The acute compound toxicity for the different routes of administration ($LD_{50}$) is determined in advance.

The test for interferon-inducing activity in humans requires serum samples to be taken firstly immediately before and then at the 24th and 48th hour after a single oral administration of 100 mg of dipyridamole in order to determine the interferon titre.

The interferon titre is determined by the following methods: by the plaque-inhibition method in L cells or in human embryonal diploid firboblasts, vesicular stomatitis virus (Indiana strain) being employed as a detector virus; by the cytopathic effect inhibition method using human embryonal diploid fibroblasts. The

titre is expressed in IU/ml by comparing with reference preparations of mouse or human interferon.

The interferon induced by dipyridamole is indentifiable by a series of tests: test for absence of virocidal effect, test for absence of antiviral specificity, test for species specificity, test for resistance at pH 2.0, heat resistance test (30—60 min at 56°C, 65°C and 75°C), trypsin sensitivity, sensitivity to ribonuclease and deoxyribonuclease and interferon neutralization test using anti-interferon serum and 1, 2 and 4 IU of the interferon tested and reference preparations.

The antiviral effect of dipyridamole as an interferon inducer *in vivo* has been proved in experimental infections with Semliki forest virus, herpes simplex virus type 1 and influenza virus A in white mice (10—12 g). Ten $LD_{50}$ of Semliki forest virus or herpes simplex virus 1 were inoculated. The dipyridamole is administered once intraperitoneally or orally (24 hours before, 24 hours after or 72 hours after the viral inoculation). The dynamics of the mortality cumulative percentage (alphaviral or herpes encephalitis) is followed as compared with the controls (placebo).

It was found that dipyridamole manifests clear interferon-inducing activity in lymphoid cells. The maximum interferon titre induced in mouse peritoneal leucocytes under the effect of the optimum interferon-inducing concentration (100 μM) exceeds 256 IU/ml.

Dipyridamole also induces interferon production in non-lymphoid human or mouse cell cultures (human embryonal diplooid fibroblasts, mouse embryonal fibroblasts, L cells), the induced titres in L cells exceeding 128 IU/ml. Data summarising the interferon-inducing activity of dypridamole *in vitro* are set out hereinafter in Table 1, the main quantitative parameters: minimum interferon-inducing concentration (MinINF—IC), optimum interferon-inducing concentration (OpINF—IC) and maximum interferon titre induced by OptINF—IC being set out. The satisfactory selectivity of this dipyridamole effect is to be noticed: the ratio between the maximum cell tolerated concentration and MinINF—IC for mouse peritoneal leucocytes is over 100; for L-cells it is over 30 and for human diploid fibroblasts over 30.

Dipyridamole has marked interferon-inducing activity *in vivo*. In white mice this compound is especially efficacious when orally administered in single doses in the range of 1—100 mg/kg. Significant levels of serum interferon (128—256 IU/ml) are detected as early on as the 12th hour. Maximum titres are detected at 24—48 hours: 4096—8192 IU/ml. Although a reduction in interferon level is noticeable after the 72nd hour, marked interferon levels in the blood are still present as late as the 120th hour.

Dipyridamole also induces interferon when intraperitoneally or intravenously administered. In these cases the effective dose amounts to 0/1—16.7 mg/kg or more, and 0.01—50 mg/kg, respectively, although the maximum titres achieved are considerably lower: 256 and 128 IU/ml for the optimum doses of 16.7 and 0.1 mg/kg, respectively.

The interferon-inducing ability of dipyridamole is characterised by well-expressed selectivity. In oral administration, the single (acute) $LD_{50}$ of the compound is 2150 mg/kg, i.e. the selectivity index is in the range of 21.5—689. In intravenous administration, the single $LD_{50}$ is 150 mg/kg, i.e. the selectivity index in that case also reaches quite high values of up to 1500.

Dipyridamole, taken by human volunteers orally in single 100 mg doses (1—2 mg/kg) has been found to stimulate markedly interferon production in 80% of the treated subjects. An increase in the average geometrical titres of interferon in the blood of over 200 times is recorded after 24—48 hours in comparison with the initial level from 5.2 ± 1.7 IU/ml initial level, to 1069.0 ± 479.7 IU/ml.

Interferon, induced in cell cultures and present in the blood serum after dipyridamole administration, may be identified by means of complex physicochemical and biological tests and by means of immunological test for neutralization by anti-interferon sera (globulins) like IFN-α and IFN-β.

When employed in single doses corresponding to the interferon-indicing ones, dipyridamole leads to marked antiviral activity with respect to viral infections *in vivo*. In experiments with test animals (white mice) infectious with influenze virus A, Semliki forest virus and herpes simplex virus 1, a marked degree of protection is recorded when the dipyridamole was administered orally or intraperitoneally in single doses 24 hours before, 24 or 72 hours after virus inoculation.

When employed, dipyridamole can be applied either in powder form or granulated (tablets), as aqueous solutions, emulsions or suspensions.

The advantages of the interferon induction method of this invention which show it to be superior to other methods using interferon inducers may be summarised as follows:

*In vivo* (warm-blooded animals)

— induction of high interferon titre in the organism;

— convenient administration route — the highest interferon titres are achieved in oral administration;

— quite low inducer toxicity, relative high selectivity of the interferon-inducing effect; this is a characteristic which with dipyridamole is markedly in excess of that achieved with prior art low-molecular weight inducers as well as practically all the high molecular interferon inducers;

— substantially continuous preservation of the high interferon levels in the organism, in the serum even 96 hours after administration of the inducer; and

*In vitro*

— clear interferon induction in cultures both of lymphoid cells (explanted, lines) and in non-lymphoid cells (fibroblasts, etc) in good yields. The interferon is then purified and separated from the medium in which it has been produced; by this characteristic the dipyridamole is superior to most of the well-known low-molecular weight interferon inducers.

The following Examples illustrate this invention:

Example 1

Dipyridamole interferon-inducing activity was determined in different types of cell cultures. Maximum interferon titres exceeding 256 IU/ml were produced in mouse peritoneal leucocytes, explanted *in vitro*, under the effect of 100 μMl of dipyridamole. This titre is higher than that induced by double-stranded RNA of phage f2 of E. coli (5 μg/ml). In monolayer L cell culture containing the dipyridamol in 30 μMl concentrations, interferon was induced to an extent of 128 IU/ml. This exceeds considerably the interferon production induced by poly I:C (10 μg/ml). The results obtained are set out in Table 1.

TABLE 1

Interferon-inducing Activity of Dipyridamole in Cell Cultures

| Cellular Culture | MinIFN—IC micromol/l | OptINF—IC Micromol/l | Maximum Interferon titre IU/ml |
|---|---|---|---|
| Mouse peritoneal leucocytes | 3 | 100—1000 | ≥256 |
| L cells | 10 | 30—300 | ≥128 |
| Mouse embryonal fibroblasts | 100 | 300 | 16 |
| Human diploid embryonal fibroblasts | 10 | 100—300 | 16—32 |

Example 2

Table 2 and Figures 1 and 2 of the accompanying drawings illustrate the interferon-inducing activity of dipyridamole in white mice using different administration routes: intravenously (Table 2), intraperitoneally (Fig. 1) and orally (Fig. 2).

TABLE 2

Serum Interferon Level in White Mice
after Intravenous Administration of Dipyridamole

| Dypyridamole dose mg/kg | Interferon Titres (IU/ml) at different time intervals after Dipyridamole Administration | | | | | |
|---|---|---|---|---|---|---|
| | hours: | 6th | 12th | 24th | 48th | 72nd |
| 50.0 | | | 8 | 8(4) | | |
| 16.7 | | | 32 | 16 | | |
| 5.5 | | | 16 | 16 | | |
| 1.8 | | | 16 | 16 | | 8 |
| 0.6 | | <4 | 8 | 32 | 16 | 16 |
| 0.2 | | | | ≤4 | 32 | 32 |
| 0.1 | | ≤4 | >4 | ≤4 | 128 | 32 |
| 0.05 | | | | ≤4 | 64 | 32 |
| 0.01 | | | | | | 16 |
| 0 (placebo) | | <4 | <4 | <4 | <4 | 4 |

Following intravenous administration of 0.1 mg/kg, the highest titres were reached relatively late: at the 48th hour (128 IU/m). Higher doses induced maximum interferon levels at the 12—24th hour.

When administered intraperitoneally in a 16.7 mg/kg dose as can be seen from Fig. 1, the dipyridamole induced peak levels of 256 IU/ml in blood at the 24—48th hour after the inoculation. Significant concentrations were detected until the 120th hour. Lower doses, below 0.1 mg/kg all showed lower but marked activity (Fig. 1).

Dipyridamole shows maximum interferon-inducing activity after oral administration (Fig. 2). Serum interferon levels of 128—256 IU/ml were established as early on as on the 12th hour when employing single doses in the 6.25—100 mg/kg range. Peak values in the range of 4096—8192 IU/ml were reached at the 48th hour after the administation of 12.5 or 25 mg/kg. The interferon titres remained higher than those in the control (placebo) group as late as the 120th hour (Fig. 2).

Example 3

Table 3 illustrates the inteferon-inducing activity of dipyridamole in healthy volunteer subjects. A significant raising of the serum interferon level was established in 80% of subjects at the 24th and 48th hour on administering 100 mg in single dose orally to a group of 20 men and women aged between 30 and 35.

TABLE 3

Mean Values of Serum Interferon Titres in Human Volunteers Having Positive Response

| Time after dipyridamole administration | Interferon titre IU/ml |
|---|---|
| 0 hours | 5.2 ± 1.7 |
| 24—48 hours | 1069.0 ± 479.7 |

Example 4

Tables 4 and 5 show the properties of interferon which has been induced *in vitro* and *in vivo* by dipyridamole.

TABLE 4

Characteristics of Interferon Induced Using Dipyridamole

| | | Interferon Titre (IU/ml) | | |
|---|---|---|---|---|
| Treatment | Time | L cells | Mouse peritoneal leucocytes | Mouse Serum |
| Controls | | 16 | 32 (16) | 128 |
| pH 2.0 | 18 h | 16 | 32 | 128 |
| 56°C | 30 min | 8 (4) | 16 | 128 |
| | 60 min | 4 | 8 | 8 |
| 65°C | 30 min | 4 | 8 (16) | 4 |
| 75°C | 30 min | 4 | <4 | |
| Trypsin | | <4 | <4 | <4 |
| Ether | | 16 (8) | 32 | |
| Ribonuclease | | 16 | 32 | 28 |
| Deoxyribonuclease | | 16 | 32 | 128 |

TABLE 5

Neutralizaton of Dipyridamole-induced Mouse Interferon by Anti-interferon Serum Globulin

| Interferon preparation | IU in neutralisaton test mixture | Final dilution of anti-interferon serum globulin neutralizing interferon activity |
|---|---|---|
| Reference mouse interferon +) | 4 | 1:8192 |
| | 2 | 1:16384 |
| | 1 | 1:>65536 |
| Dipyridamole induced mouse interferon ++) | 4 | 1:>32 |
| | 2 | 1:>1024 |
| | 1 | 1:>1024 |

+) induced in L cells
++) serum samples of mice inoculated intravenously with dipyridamole

Example 5

Tables 6 and 7 illustrate the antiviral effect achieved in experimental Semliki forest virus and herpes simplex virus infections in white mice to which dipyridamole has been administered in single dose intraperitoneally 24 hours before the viral inoculation. Following oral administration the effect is much stronger.

TABLE 6

Dipyridamole (+) Effect in Semliki Forest Virus Experimental Infection in White Mice (5—10 $LD_{50}$)

| Dipyridamole dose mg/kg | Mortality (++) No. % M±m | t | Protection coefficient | Protection index % Activity | Mean survival time: days |
|---|---|---|---|---|---|
| 1.8 | 3/10 30.0±15.27 | 3.02 | 2.4 | 5.8.0 ++ | 22.6±3.77 |
| 0.1 | 4/10 40.0±16.32 | 2.64 | 1.8 | 44.0 + | 20.7±3.82 |
| Placebo | 7/10 70.0±15.27 | | | | 12.9±2.91 |

(+) Single administration intraperitoneally 24 hours before viral inoculation
(++) Followed until the 30th day after infection.

TABLE 7

Dipyridamole (+) Effect in Herpes Simplex 1 Virus, Lennett Strain (10 $LD_{50}$) in Experimental Infection

| Dipyridamole dose mg/kg | Mortality (++) No. % M±m | t | Protection coefficient | Protection index % Activity | Mean survival time: days |
|---|---|---|---|---|---|
| 16.7 | 11/20 55.0±11.41 | 3.94 | 1.81 | 44.75 ++ | 17.3±2.69 |
| Placebo | 19/20 95.0±5.0 | | | | 6.3±1.29 |

(+) Single administration intraperitoneally 24 hours before viral inoculation
(++) Followed until the 30th day after infection

**Claims**

1. A method of inducing the production of interferon by cellular material which is characterised by administering the compound dipyridamole to cellular material held in vitro and on which dipyridamole does not have a biocidal effect, retaining the dipyridamole in contact with the cellular material for a period

of time sufficient for interferon production to be induced and isolating interferon from the medium in which it had been produced.

2. A method as claimed in claim 1, wherein the cellular material is a lymphoid cell culture or comprises non-lymphoid cells from humans or mice.

3. A method as claimed in claim 2, wherein the non-lymphoid cells are human embryonal diploid fibroblasts or mouse embryonal fibroblasts or L cells.

4. A method for the production of interferon which is characterised by administering the compound dipyridamole in an interferon inducing single dose to a non-human warm-blooded animal, removing serum from the animal and separating interferon from the serum.

5. A method as claimed in claim 4, wherein said compound is administered orally to said animal.

6. A method as claimed in claim 4 or 5, in which said compound is supplied in a single dose of 1.0 to 100 mg/kg.

7. A method as claimed in claim 4, in which said compound is administered intraperitoneally in an amount of 0.01 to 16.7 mg/kg.

8. A method as claimed in claim 4, in which said compound is administered intravenously in an amount of from 0.01 to 50 mg/kg.

9. Use of the compound dipyridamole for the manufacture of a medicament for the induction of interferon in a warm-blooded animal to have an antiviral effect therein.

10. Use of the compound dipyridamole for the manufacture of an orally administrable medicament for the induction of interferon in a warm-blooded animal to have an antiviral effect therein.

11. The use of the compound dipyridamole according to claim 10 for the manufacture of a single dosage unit of the medicament, which single dosage unit is appropriate to the warm-blooded animal and contains from 1 to 100 mg of dipyridamole per kg of animal weight.

12. Use of the compound dipyridamole for the manufacture of an intraperitoneally administrable medicament for the induction of interferon in an warm-blooded animal to have an antiviral effect therein, which medicament is in the form of a single dosage unit appropriate to the warm blooded animal and contains from 0.01 to 16.7 mg of dipyridamole per kg of animal weight.

13. Use of the compound dipyridamole for the manufacture of an intravenously administrable medicament for the induction of interferon in a warm-blooded animal to have an antiviral effect therein, which medicament is in the form of a single dosage unit appropriate to the warm-blooded animal and contains from 0.01 to 50 mg of dipyridamole per kg of animal weight.

**Patentansprüche**

1. Verfahren zur Induzierung der Produktion von Interferon durch Zellmaterial, gekennzeichnet durch die Einführung der Verbindung Dipyridamol in das in vitro gehaltene Zellmaterial, wobei das Dipyridamol keine biozide Wirkung aufweist, Halten des Dipyridamols im Kontakt mit dem Zellmaterial während einere für die zu induzierende Interferonproduktion ausreichenden Zeitdauer und Isolierung des Interferons aus dem Medium, in dem es erzeugt worden ist.

2. Verfahren nach Anspruch 1, worin das Zellmaterial eine Lymphoidzellenkultur ist oder Nicht-Lymphoidzellen von Menschen oder Mäusen umfaßst.

3. Verfahren nach Anspruch 2, worin die Nicht-Lymphoidzellen menschliche embryonale diploide Fibroblasten oder embryonale Fibroblasten von Mäusen oder L-Zellen sind.

4. Verfahren zur Herstellung von Interferon, das dadurch gekennzeichnet ist, daß man die Dipyridamolverbindung in einer Interferon induzierenden Einzeldosis einem nichtmenschlichen Warmblüter verabreicht, das Serum aud dem Tier entfernt und aus dem Serum das Interferon abtrennt.

5. Verfahren nach Anspruch 4, worin die Verbindung dem Tier oral verabreicht wird.

6. Verfahren nach Anspruch 4 oder 5, worin die Verbindung in einer einzigen Dosis von 1,0 bis 100 mg/kg zugeführt wird.

7. Verfahren nach Anspruch 4, worin die Verbindung intraperitoneal in einer Menge von 0,1 bis 16,7 mg/kg verabreicht wird.

8. Verfahren nach Anspruch 4, worin die Verbindung intravenös in einer Menge von 0,01 bis 50 mg/kg verabreicht wird.

9. Verwendung der Verbindung Dipyridamol zur Herstellung eines Medikaments für die Induzierung des Interferons in einem Warmblüter zur Erzeugung einer antiviralen Wirkung.

10. Verwendung der Verbindung Dipyridamol zur Herstellung eines oral verabreichbaren Medikaments für die Induzierung des Interferons in einem Warmblüter zur Erzeugung einer antiviralen Wirkung.

11. Verwendung der Verbindung Dipyridamol nach Anspruch 10 zur Herstellung einer Einzeldosiseinheit des Medikaments, wobei die Einzeldosiseinheit für den Warmblüter geeignet ist und 1 bis 100 mg Dipyridamol/kg Gewicht des tieres enthält.

12. Verwendung der Verbindung Dipyridamol zur Herstellung eines intraperitoneal verabreichbaren Medikaments für die Induzierung des Interferons in einem Warmblüter zur Erzeugung einer antiviralen Wirkung, wobei das Medikament in Form einer für den Warmblüter geeigneten Einzeldosiseinheit vorliegt und 0,01 bis 16,7 mg Dipyridamol/kg Gewicht des Tieres enthält.

13. Verwendung der Verbindung Dipyridamol zur Herstellung eines intravenös verabreichbaren

Medikaments für die Induzierung des Interferons in einem Warmblüter zur Erzeugung einer antiviralen Wirkung, wobei das Medikament in Form einer für den Warmblüter geeigneten Einzeldosiseinheit vorliegt und 0,01 bis 50 Dipyridamol/kg Gewicht des Tieres enthält.

**Revendications**

1. Procédé pour induire la production d'interféron par un matériel cellulaire, qui est caractérisé par:

l'administration du composé dipyridamole au matériel cellulaire maintenu *in vitro* et sur lequel la dipyridamole n'a pas d'effet biocide:

le maintien de la dipyridamole en contact ave le matériel cellulaire pendant une période de temps suffisante pour que la production d'interféron soit induite; et

l'isolement de l'interféron à partir du milieu dans lequel il a été produit.

2. Procédé selon la revendication 1, dans lequel le matériel cellulaire est une culture de cellules lymphoïdes ou comprend des cellules non-lymphoïdes prélevées sur l'homme ou la souris.

3. Procédé selon la revendication 2, dans lequel les cellules non-lymphoïdes sont des fibroblastes diploïdes embryonnaires humains ou des fibroblastes embryonnaires de souris ou des cellules L.

4. Procédé pour la production d'interféron, qui comprend:

l'administration du composé dipyridamole, à une dose unique inductrice d'interféron, à un animal à sang chaud non-humain;

le prélèvement du sérum de l'animal; et

la séparation de l'interféron à partir du sérum.

5. Procédé selon la revendication 4, suivant lequel on administre ledit composé audit animal par la voie orale.

6. Procédé selon la revendication 4 ou 5, suivant lequel on administre ledit composé à une dose unique allant de 1,0 à 100 mg/kg.

7. Procédé selon la revendication 4, suivant lequel on administre ledit composé par la voie intrapéritonéale en une quantité allant de 0,01 à 16,7 mg/kg.

8. Procédé selon la revendication 4, suivant lequel on administre ledit composé par la voie intraveineuse en une quantité allant de 0,01 à 50 mg/kg.

9. Utilisation du composé dipyridamole pour la fabrication d'un médicament pour l'induction d'interféron chez un animal à sang chaud, pour obtenir un effet antiviral chez ce dernier.

10. Utilisation du composé dipyridamole pour la fabrication d'un médicament administrable par la voie orale, pour l'induction d'interféron chez un animal à sang chaud, pour obtenir un effet antiviral chez ce dernier.

11. Utilisation du composé dipyridamole selon la revendication 10, pour la fabrication d'une unité de dosage unique du médicament, cette unité de dosage unique convenant pour un animal à sang chaud et contenant de 1 à 100 mg de dipyridamole par kg de poids de l'animal.

12. Utilisation du composé dipyridamole pour la fabrication d'un médicament administrable par la voie intrapéritonéale, pour l'induction d'interféron chez un animal à sang chaud, pour obtenir un effet antiviral chez ce dernier, ce médicament se présentant sous la forme d'une unité de dosage unique qui convient pour l'animal à sang chaud, et contient de 0,01 à 16,7 mg de dipyridamole par kg de poids de l'animal.

13. Utilisation du composé dipyridamole pour la fabrication d'un médicament administrable par la voie intraveineuse, pour l'induction d'interféron chez un animal à sang chaud, pour obtenir un effet antiviral chez ze dernier, ce médicament se présentant sous la forme d'une unité de dosage unique qui convient pour l'animal à sang chaud, et contient de 0,01 à 50 mg de dipyridamole par kg de poids de l'animal.

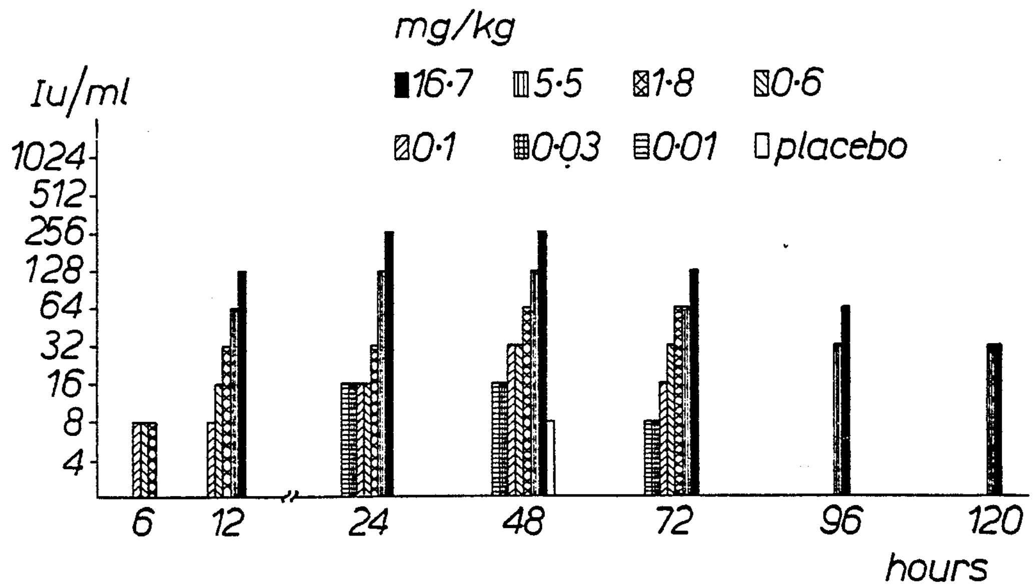
mg/kg
16·7
5·5
1·8
0·6
0·1
0·03
0·01
placebo
Iu/ml
1024
512
256
128
64
32
16
8
4
6
12
24
48
72
96
120
hours

FIG. 1.

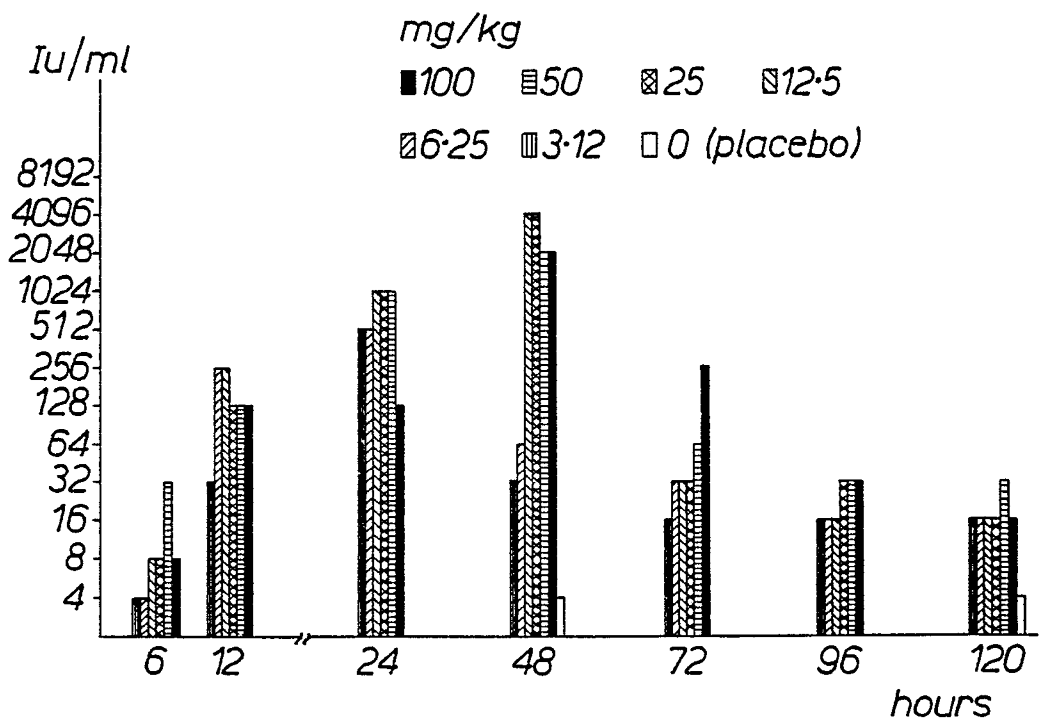
mg/kg
100
50
25
12·5
6·25
3·12
0 (placebo)
Iu/ml
8192
4096
2048
1024
512
256
128
64
32
16
8
4
6
12
24
48
72
96
120
hours

FIG. 2.